# EUROPEAN PATENT SPECIFICATION

(11) **EP 1 314 413 B1**
(45) Date of publication and mention of the grant of the patent: **10.06.2009**
(21) Application number: 02258088.0
(22) Date of filing: 25.11.2002
(51) Int. Cl.: A61F 13/472

(54) **A sanitary napkin**
Damenbinde
Serviette hygiénique

(30) Priority: 26.11.2001 BR 0105724
(43) Date of publication of application: 28.05.2003
(73) Proprietor: Johnson & Johnson Industrial Ltda., Sao Jose dos Campos, SP (BR)
(72) Inventor: De Carvalho, Antonio Carlos Ribeiro, Tabaute, Sao Paulo (BR); Fajolli, Marcia Helena Teixeira, 12242-032 Sao Jose Dos Campos, Sao Paulo (BR)
(74) Representative: Mercer, Christopher Paul

(56) References cited:
- WO-A-99/25290
- DE-U- 29 600 384
- DE-U- 29 911 806
- US-A- 1 946 626
- US-A- 3 430 630
- US-A- 4 184 498
- US-A- 4 900 319

## Description

The present invention relates to a sanitary napkin for absorbing bodily exudates, such as menstruation liquids and urine, enhancing the wearer's comfort.

### Background of the Invention

It is well-known that conventional sanitary napkins have a substantially planar elongate shape. Although many of the more ancient sanitary napkins are substantially rectangular in shape, having straight side edges, a number of more recent sanitary napkins have been molded so as to improve the adjust of the contours of the body of a wearer, for instance in the form of an hourglass. These hourglass-shaped sanitary napkins have absorbent portions, the maximum breadth of which is located in the end regions of the product, that it to say, generally up to 1/6 of its length as measured inwards from the distal end of the sanitary napkin. However, it has been found that these sanitary napkins have a number of drawbacks, which are described below.

In the first place, these sanitary napkins are inefficient, since they have a larger amount of absorbent material in the end regions of the sanitary napkin, which are regions in which leakage hardly occurs, and a smaller amount of absorbent material in a central region, which is adapted to be located between the wearer's thighs.

Secondly, the excess material in the end regions and the absence of material in the region between the thighs cause a deformation in the sanitary napkin, due to this accumulation of material, which causes discomfort in use and may lead to leakage of the bodily exudate.

### Summary of the Invention

An objective of the present invention is to provide a sanitary napkin having an end portion that is substantially thin, provides an absorption capacity comparable with that of conventionally molded sanitary napkins and brings about excellent comfort in use.

The objectives of the present invention are achieved by means of a sanitary napkin comprising an absorbent portion that has a first transverse end and a second opposite transverse end, which define a length between themselves, a first longitudinal side edge and a second opposite longitudinal side edge, which define a breadth between themselves, the breadth varying along the length of the absorbent portion, wherein:
(i) a first breadth is located between 1/5 and less than ½ of the length of the absorbent portion, measured from the first transverse end, the first breadth being larger than any breadth of the absorbent portion located between the first transverse end and the first breadth;
(ii) a second breadth located substantially from 1/5 to less than ½ of the length of the absorbent portion, as measured from the second transverse end, the second breadth being smaller then or equal to the first breadth;
(iii) a central region intermediate between the first breadth and the second breadth, the central region having a maximum breadth that is smaller then or equal to the first breadth.

The sanitary napkin is characterized in that the absorbert portion has a plurality of elongate grooves, each groove being spaced from an adjacent groove and extending obliquely with respect to a longitudinal axis from one side of the sanitory napkin to the opposite side of the sanitory napkin.

### Brief Description of the Drawings

The present invention will now be described in greater details with reference to an embodiment represented in the drawings. The figures show:
- Figure 1 is a perspective view of an embodiment of a sanitary napkin not in accordance with the present invention;
- Figure 2 is a top view of the sanitary napkin illustrated in Figure 1;
- Figure 3 is a bottom view of the sanitary napkin illustrated in figures 1 and 2;
- Figure 4 is a perspective view of a first embodiment of the sanitary napkin of the present invention:
- Figure 5 is a top view of the sanitary napkin illustrated in Figure 4;
- Figure 6 is a bottom view of the sanitary napkin illustrated in Figures 4 and 5;

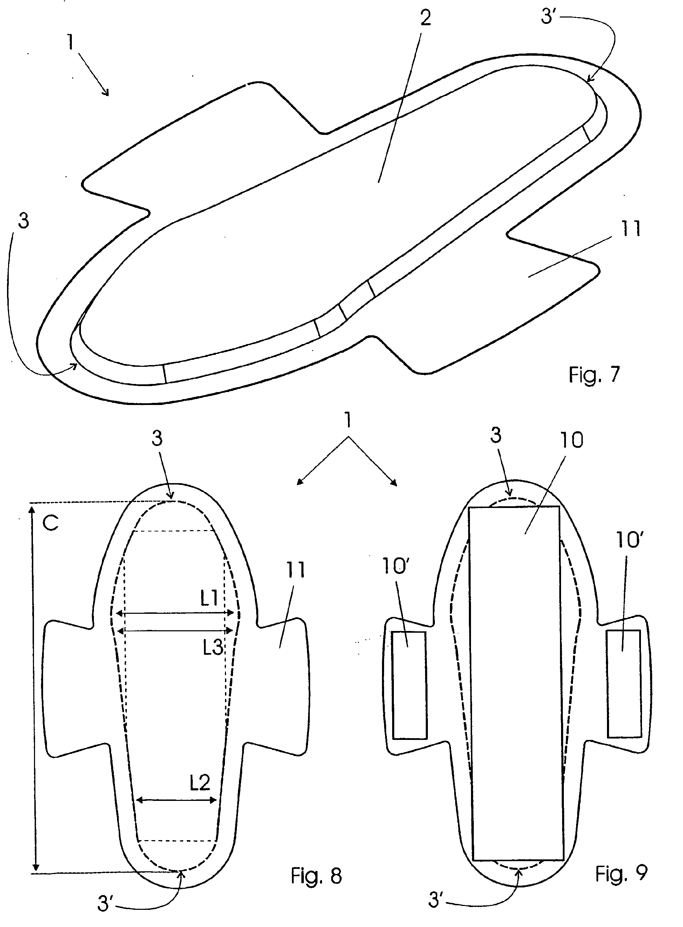

- Figure 7 is a perspective view of an embodiment of a sanitary napkin not in accordance with the present invention;
- Figure 8 is a top view of the sanitary napkin illustrated in figure 7;
- Figure 9 is a bottom view of the sanitary napkin illustrated in Figures 7 and 8;

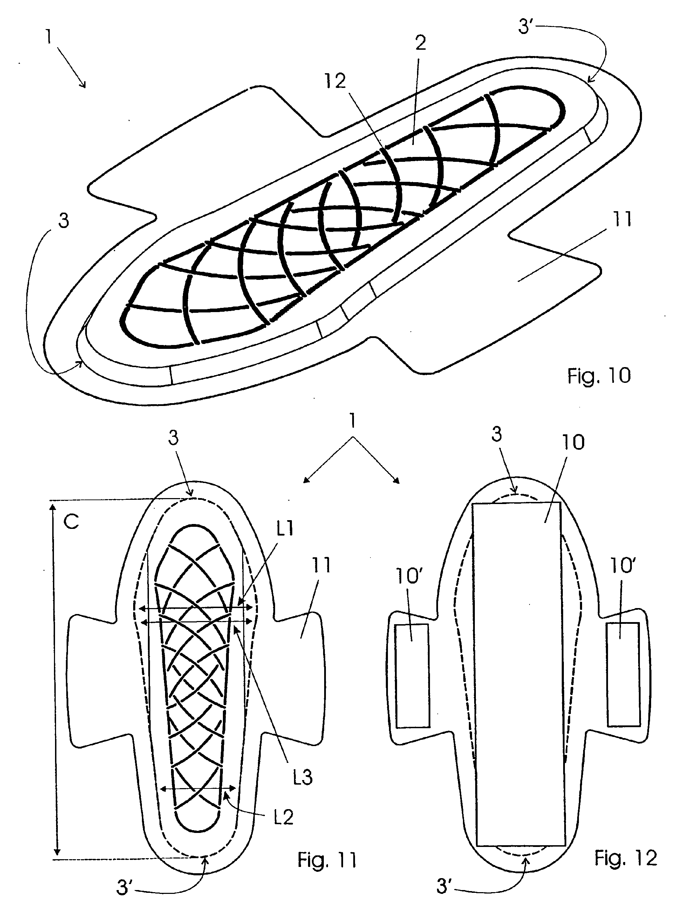

- Figure 10 is a perspective view of a second embodiment of the sanitary napkin of the present invention;
- Figure 11 is a top view of the sanitary napkin illustrated in Figure 10;
- Figure 12 is a bottom view of the sanitary napkin illustrated in Figures 10 and 11;

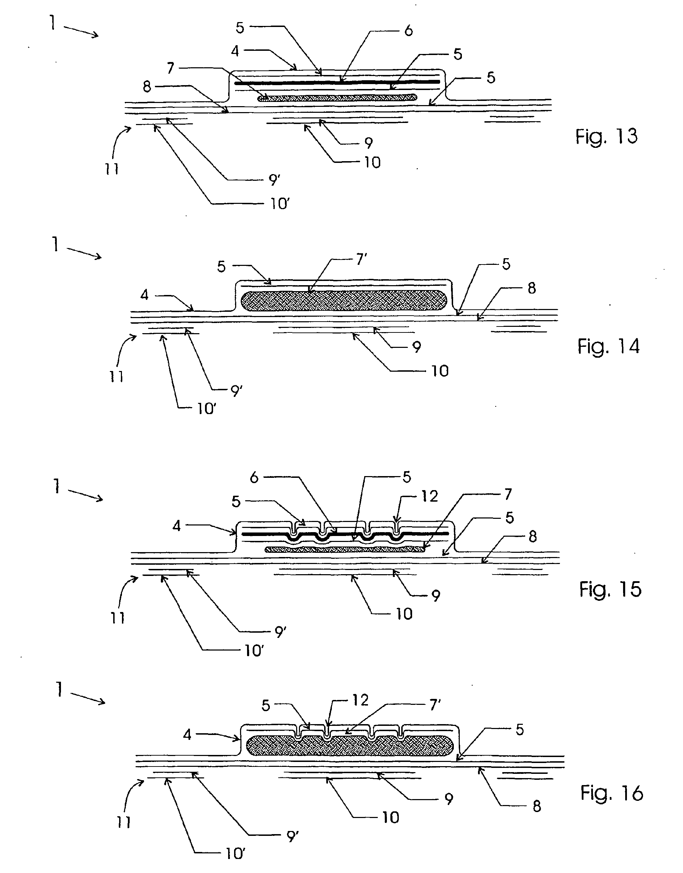

- Figure 13 is a schematic cross-section view of a first version of a sanitary napkin not in accordance with the present invention;
- Figure 14 is a schematic cross-section view of a second version of a sanitary napkin not in accordance with the present invention;
- Figure 15 is a schematic cross-section view of a first version of the first and second embodiments of the sanitary napkin of the present invention;
- Figure 16 is a schematic cross-section view of a second version of the first and second embodiments of the sanitary napkin of the present invention.

### Detailed Description of the Invention

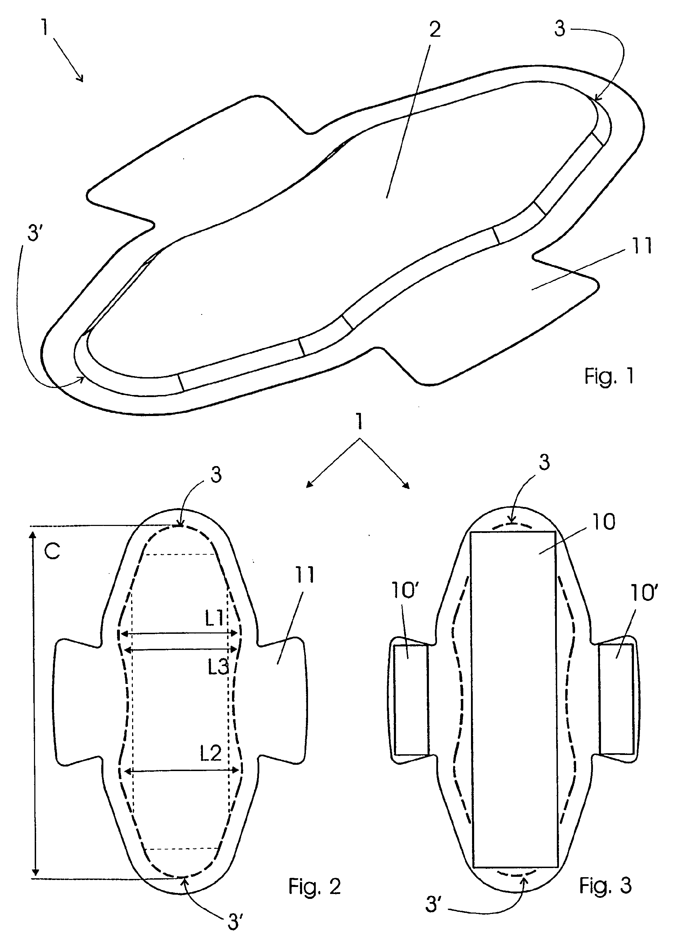

According to an embodiment of a sanitary napkin not in accordance with the present invention, as shown in Figure 1, the sanitary napkin 1 comprises a substantially planar and elongate absorbent portion 2, which forms a main body or chassis of the sanitary napkin. The absorbent portion 2 comprises a layer of liquid-permeable material, a layer of liquid-impermeable material and an absorbent core between the liquid-permeable layer and the liquid-impermeable layer. The absorbent portion 2 comprises a transverse main end 3, from which it extends as far as a second transverse main end 3'. The absorbent portion 2 also has a first longitudinal side edge and a second opposite longitudinal side edge.

The total length C of the absorbent portion 2 is the distance between the first main end 3 and the second main end 3'. The absorbent portion 2 has a breadth that varies along its length. The breadth is measured as being the distance between the first longitudinal side edge and the second longitudinal side edge along a line that is perpendicular to an imaginary longitudinal central line, that is to say, it is substantially perpendicular to the length C of the absorbent portion 2. The length C has a value greater than that of any breadth or the absorbent portion 2, which are described below.

The absorbent portion 2 has a maximum breadth L1 (hereinafter indicated as a first breadth) located between 1/5 and ½ of the value of the length C of the absorbent portion 2 measured from the first main end 3, and preferably this breadth L1 is located at about 1/3 of said value of the length C. The value of L1 is greater than that of any breadth of the absorbent portion 2 in the region between the first transverse end 3 and the first breadth L1. The region between L1 and the first transverse end 3 is smaller than L1 and, preferably, tapers in a linear way from L1 to the first transverse end 3, as shown in Figure 1.

Consequently, the breadth of the absorbent portion 2 increases between the firs main end 3 (where this value is zero) and the distance between 1/5 and ½ of its longitudinal length, where the breadth is maximum. Preferably, but not compulsorily, the breadth increases linearly with respect to the increase of the distance between the first transverse end 3 and the distance between 1/5 and ½ of the longitudinal length C. The absorbent portion 2 comprises a second breadth L2 located between 1/5 - ½ of the value of the length C of the absorbent portion 2, measured from the second transverse end 3. The value 1f L2 is smaller that or equal to the value of L1. The value of L2 is greater than any breadth of the absorbent portion 2 between the second transverse end 3 and the distance between 1/5 and ½ of its longitudinal length C. The absorbent portion 2 also has a central region intermediate between the first breadth L1 and the second breadth L2, the central region having a maximum breadth L3 that is smaller than or equal to the first breadth L1. In a preferred embodiment, L1 and L2 are substantially equal, L3 is smaller than L1 and L2, and the longitudinal side edges of the absorbent portion 2 in the central region are arcuate, in a convex orientation inwards, to form an hourglass-shaped structure. This means that the end edges of the absorbent portion 2 are substantially curve it its central region, the centers of the radii of curvature of the edges are located substantially out of the absorbent portion 2, in such a way that this central region is substantially anatomic, bringing about an optimized fitting into the region between the thighs of the wearer with a smooth movement of her thighs, providing comfort in use and the feeling of not being wearing the sanitary napkin.

This constructive arrangement characterizes a more anatomical central region, and the narrower ends result in less deformation of the product in use by virtue of the substantial reduction of material in these regions, which brings about the required protection with more comfort and discretion. This characteristic may be present either at the two ends or at the first transverse end 3 of the sanitary napkin 1.

In the case of the two ends of the absorbent portion 2 with this geometric construction, the first and the second main ends 3, 3' are indistinct, and the values of the length of the portion 2 may be taken from any one of these ends 3, 3'.

A first version of the sanitary napkin 1 comprises a layer of liquid-impermeable material 4, the inner surface of which is joined by the construction adhesive 5 to a first surface of a transfer layer 6, the second surface of which, in turn, is joined to one first surface of an absorbent core 7 (which is capable of absorbing liquid exudates), the second surface of which is joined to a first surface, or to the inner surface, of a layer of liquid-impermeable material as a liquid-impermeable plastic film 8, also by means of the construction adhesive 5. Any conventional liquid-permeable materials, liquid-impermeable materials, transfer layers and absorbent materials that are well known from the prior art are suitable for use in the present invention.

The liquid-permeable layer 4 may be formed from any flexible liquid-permeable material that does not cause irritation to an wearer. The suitable liquid-permeable materials include but are not limited to woven fabrics, non-woven fabrics, plastic films with openings and the like. The liquid-permeable layer 4 is preferably a high-transposition non-woven mesh material, bulky and of relatively low density. The liquid-permeable layer may be composed either by a single type of fiber such as polyester or polypropilene or by 2-component or conjugated fibers, which have one component of low melting point and one component of high melting point. These fibers may be selected from a variety of natural or synthetic materials such as nylon, polyester, rayon (in combination with other fibers), cotton, acrylic fiber or others, and combinations thereof.

The liquid-impermeable layer 4 preferably has a relatively high degree of wettability, although the individual fibers used for forming this layer may not be particularly hydrophilic. The liquid-permeable material of the layer should also contain a great number of relatively large pores. This occurs because the liquid-permeable layer should absorb the bodily fluid rapidly and carry it away from the body and from the deposition point. Advantageously, the fibers that compose the liquid-impermeable layer should not lose their physical properties when they are wetted. In other words, they should not collapse or lose their resiliency when they are subjected to water or to the bodily fluid. The liquid-permeable layer may be treated so as to allow the fluid to pass through it immediately. The liquid-permeable layer also acts to transfer the liquid rapidly to the other layers of the absorbent system. In this way, the liquid-permeable layer is advantageously wettable, hydrophilic and porous. When it is composed of synthetic hydrophobic fibers such as polyester fibers or 2-component fibers, the liquid-permeable layer may be treated with a surfactant in order to impart the desired degree of wettability. An alternative would be to compose the liquid-permeable layer with a film having openings.

The liquid-permeable layer may be fixed, for instance, by embossing, to the rest of the absorbent system by fixing the liquid-permeable layer to the underlying layer, in order to aid in transporting fluid from the liquid-permeable layer to the absorbent system. This fixation may be effected in the place, in a plurality of places or throughout the contact surface of the absorbent system of the liquid-permeable layer. The exemplified means of fixing the liquid-permeable layer to the absorbent system are adhesion and fusion.

The transfer layer 6 may be composed of fibrous materials such as wood pulp, polyester, rayon, or the like, or combinations thereof. In a preferred embodiment, the transfer layer 6 is composed of fibrous materials and may include thermoplastic fibers for the purpose of stabilizing and maintaining its structural integrity. The transfer layer 6 may be treated with the surfactant on one of on both sides, in order to increase its wettability, although, in general, the transfer layer 6 is relatively hydrophilic and may not need any treatment. The transfer layer 6 is preferably joined, on both sides, to the adjacent layers, that is to say, the liquid-permeable layer and an underlying liquid-impermeable layer.

The materials particularly suitable for use in the transfer layer 6 have an open cell structure that is capable of absorbing the liquid rapidly and generally has a density ranging from 0.04 to 0.05 g/cm3, a base weight ranging from 80 to 110 g/m2 and a thickness ranging from less than about 1 to 3 mm. The examples of materials suitable for the transfer layer 6 are pulp joined by air, sold by Buckeye of Memphis, Tennessee, under the designation VIZORB 3008, which has a base weight of 110 g/m2, and VIZORB 3010, which has a base weight of 90 g/m2.

The absorbent core 7 is placed immediately underlying and joined to the transfer layer 6. In a preferred embodiment, the absorbent core 7 is a combination or a mixture of cellulose fibers and superabsorbent material is arranged in and between the fibers of this pulp.

In a specific example, the absorbent core 7 is a material that contains from about 40 percent by weight to about 95 percent by weight of cellulose fibers, and more specifically from about 60 to about 80 percent by weight of cellulose fibers. This material may contain from about 5 percent by weight to about 60 percent by weight of SAP (superabsorbent polymers), preferably from about 20 to about 55 percent by weight of SAP, and more preferably from 30 to 45 percent by weight of SAP, and still more preferably about 40 percent by weight of SAP. The material has water contents of less than about 10 percent by weight. As used here, the expression "percent by weight" refers to the weight of the substance by weight of the final material. For example, 10 percent by weight of SAP means 10 g/m2 of SAP per 100 g/m2 of the base weight of the material.

The cellulose fibers that may be used in the absorbent core 7 are well known from the prior art and include wood pulp, cotton, linen and turf moss. The wood pulp is preferred. Pulps may be obtained from pulp waste material, sulfite paper, mechanical or chemomechanical kraft, from organic solvent pulps, etc. Both kinds of soft wood and hard wood are useful. Soft wood pulps are preferred. It is not necessary to treat the cellulose fibers with agents that release chemicals, crosslinking agents and other similar ones for use in the present material.

The absorbent core 7 may contain any superabsorbent polymer (SAP), the SAPs being well known from the prior art. For the purposes of the present invention, the expression "superabsorbent polymer" (or "SAP") refer to the materials that are capable of absorbing and retaining at least ten times its weight in bodily fluids under a pressure of 3.447 kPa (0.5 1b. per square inch). The superabsorbent polymer particles of the invention may be of inorganic or organic cross-linked hydrophilic polymers, such as polyvinyl alcohols, polyethilene oxides, both of them being cross-linked, guar gum, xanthane, gum and others. The particles may be in the form of a powder, grains, granules or fibers. The preferred superabsorbent polymer particles for use in the present invention are cross-linked polyacrylates, such as the product offered by Sumitomo Seika Chemicals Co., Ltd., from Osaka, Japan, under the designation SA60N Type II*, the product offered by Stockhausen, Inc.1, from Greensboro, North Caroline, under the designation 7440, and the product offered by Chemdal International, Inc., from Palatine, Illinois, under the designation 2100^{A}*.

The absorbent core 7 may be manufactured by employing means such as decomposition by air. According to this process, the cellulose fibers (for example, pulp) are processed by using a hammer grinder for individualizing the fibers. The individualized fibers are mixed with SAP granules in a mixing system and pneumatically carried to a series of formation heads. The mixing and distribution of the fibers and of the SAP granules may be controlled separately for each formation head. The controlled air circulation and the stirrers provided with flaps in each chamber bring about uniform mixing and distribution of the pulp and of the SAP. The SAP may either be mixed completely or homogeneously throughout the material or be contained in the specific layers upon being distributed to the selected formation heads. The fibers (and the SAP) of each formation chamber are deposited by means of vacuum into a formation thread, thus forming an absorbent mesh in layers. The mesh is subsequently compressed by using calenders to achieve the desired density. The adequate mesh is curled by using a conventional curling equipment. The formation thread may be coated with tissue paper to reduce the loss of material. The layer of tissue paper may be removed prior to calendering or may be incorporated into the formed material. In a possible variant, the transfer layer may be formed integrally with the absorbent core 7, in order to obtain a unified absorbent system.

The absorbent core 7 is preferably of a high density and preferably has a density higher than about 0.25 g/cm³. In general, the absorbent core 7 has a density ranging from about 0.25 to about 0.50 g/cm³, more preferably from about 0.25 g/cm³ to about 0.40 g/cm³, and still more preferably from about 0.25 g/cm³ to about 0.35 g/cm³.

The absorbent placed by air are typically produced with a low density. In order to achieve higher levels of density, such as the examples of the absorbent core 7 supplied above, the material placed by air is compressed by using calenders. The compaction is carried out by using means known from the prior art. This compaction is typically carried out at a temperature of about 100°C and at a load of about 130 newtons per millimeter.

The absorbent core 7 may be prepared in a wide range of base weights. The absorbent core 7 may have a base weight ranging from about 100 g/m² to about 700 g/m². In a specific example, the vase weight varies from about 150 g/m² to about 400 g/m². Preferably, the base weight ranges from about 200 g/m² to about 350 g/m², and more preferably up to 25 g/m².

The transfer layer 6 has the function of rapidly absorbing the retaining the fluid, which is then absorbed more slowly by the absorbent core 7. The transfer layer 6, which has a structure of relatively open pores, immediately absorbs and disperses the liquid laterally within its volume and immediately transfers the liquid to the reception surface of the absorbent core. In turn, the absorbent core that has a structure of relatively smaller pores than that of the transfer layer 6, has good capillarity, which effectively attracts the liquid into its volume in the transfer layer 6. Once the liquid has been absorbed in the superabsorbent polymer, the liquid cannot be subsequently released from by application of pressure. Therefore, the liquid absorbed into the superabsorbent material remains trapped. At the same time, the force with which the absorbent core 7 consumes the liquid from the transfer layer 6 aids in reducing the amount of liquid retained in the transfer layer 6, thus reducing the amount of liquid that return to the liquid-permeable layer when the sanitary napkin is subjected to the mechanical load. In addition, the transfer layer 6 has a relatively high capillarity, so that any concentration of liquid in the transfer layer 6 that results from the mechanical load may be redistributed within the material for lesser concentrations, again reducing the amount of liquid that may return to the liquid-permeable layer.

In a specific embodiment, the absorbent core 7 contains from about 30 to 40 percent by weight of superabrobent material, has a base weight ranging from about 200 to 400 g/m2 and a density ranging from 0.2 to 0.5 g/cm³. More specifically, the density ranges from about 0.25 g/cm³ to about 0.45 g/cm³, and still more specifically it is of 0.3 g/cm³.

The absorbent core 7 may be formed as three or four blades or layers. These layers include a lower layer, one or two intermediate layers and an upper layer. The specific examples of the 3-layer or 4-layer material are indicated below. The SAP may be included in a few or all the layers. The concentration (percent by weight) of the SAP in each layer may vary, as well as the nature of the particular SAP.

Even where prepared from multiple layers, the final thickness of the formed absorbent core 7 is preferably small. The thickness may vary from less than about 0.5 mm to about 2.5 mm. In a specific example, the thickness is of less than about 0.5 mm to about 1.5 mm.

Underlying the absorbent system, there is a layer of liquid-impermeable material to prevent the liquid that is trapped in the absorbent system from coming out of the sanitary napkin and staining the wearer's undergarment. The liquid-impermeable layer is preferably made of a polymeric film, although it may be made of an air-permeable and liquid-impermeable material, such as films or foams treated with repellent or non-treated or microporous films.

The liquid-permeable layer and the liquid-impermeable layer are joined along their marginal parts, so as to form an envelope or a sealing flange that forms a unitary absorbent product and keeps the absorbent system secured. The joining may be effected by means of adhesives, heat application, ultrasound application, radiofrequency sealing, mechanical frizzing and the like, and combinations thereof.

Optionally, one flap 11 is laterally projected outwardly of each longitudinal side. The flap 11 has the shape of an isosceles trapezoid with the top adjacent to the longitudinal side and the base at the distal end. The flap 11 is preferably made as integral extensions of the liquid-permeable layer and of the liquid-impermeable layer. These integral extensions are joined to each other along their marginal seals by adhesives, heat application, ultrasound application, radiofrequency sealing, mechanical frizzing and the like, and combinations thereof. More frequently, this joining is effected at the same time as the liquid-permeable and liquid-impermeable layers are joined to each other to enclose the absorbent system. Alternatively, the flaps may include the absorbent material between the liquid-permeable layer and the extensions of the barrier layers. This absorbent material may be an extension of the transfer layer 6, of the absorbent core 7, or of both.

Finally, there is an adhesive for positioning the sanitary absorbent 1 centrally on the wearer's undergarment (not shown), positioned in a second surface, or outer surface, of the film 8, and protected by a sheet of protection material 10, which should be removed when the sanitary napkin 1 is put on. Optionally, in the example of the sanitary napkin that has the flap 11, it also comprises at least one layer of adhesive for positioning the flap covered with a sheet of protection material 10' analogous to the one described above.

A first embodiment of the sanitary napkin according to the present invention comprises a substantially thicker layer of absorbent compound 7' than that employed in the first embodiment, which occupies the place of the transfer layer 6 and the thin absorbent compound 7.

The absorbent portion 2 also comprises grooves 12, such as drawings obtained by means of the channels 12 formed throughout the absorbent core to direct the liquid along the groove (grooves) for subsequent absorption in the transfer layer 6. The grooves are formed by applying localized pressure onto the absorbent material a pressure such as, for example, that conventionally used on the relief. The applied pressure results in making the material that defines the groove floor denser, which renders it less permeable to liquid, thus extending in the distance by which the liquid may move prior to absorption. It has been found that the employ of grooves significantly contributes to the stability of the sanitary napkin in use and also in the potential of the sanitary napkin retaining the liquid in contact with the wearer's body, which is often indicated as a rewetting potential. In addition, the employ of one or more grooves adjacent to the liquid-permeable layer allows the liquid to be carried rapidly on the sanitary napkin, so that different regions of the transfer layer 6 can act efficiently to absorb the liquid in parallel. This helps in guaranteeing that the liquid will be present in a larger part of the surface area of the absorbent napkin 7, to increase the effectiveness of the absorbent core 7 in extracting the liquid from the transfer layer 6. The sanitary napkin has a plurality of elongate grooves formed in it, which are spaced from one another and configured to channel the liquid laterally through the surface of the sanitary absorbent facing the body or close to its surface part facing the body, away from the region of initial deposition.

The sanitary napkin is provided with a plurality of grooves which extend obliquely to the longitudinal axis from one side of the sanitary napkin to the other (or substantially perpendicular to the longitudinal axis). The grooves may have any shape that may be selected according to the particular application, for example, the grooves may be linear, arcuate or they may have a serpentine configuration, or a mixture thereof, as well as other shapes, including a spiral or zigzag patterns.

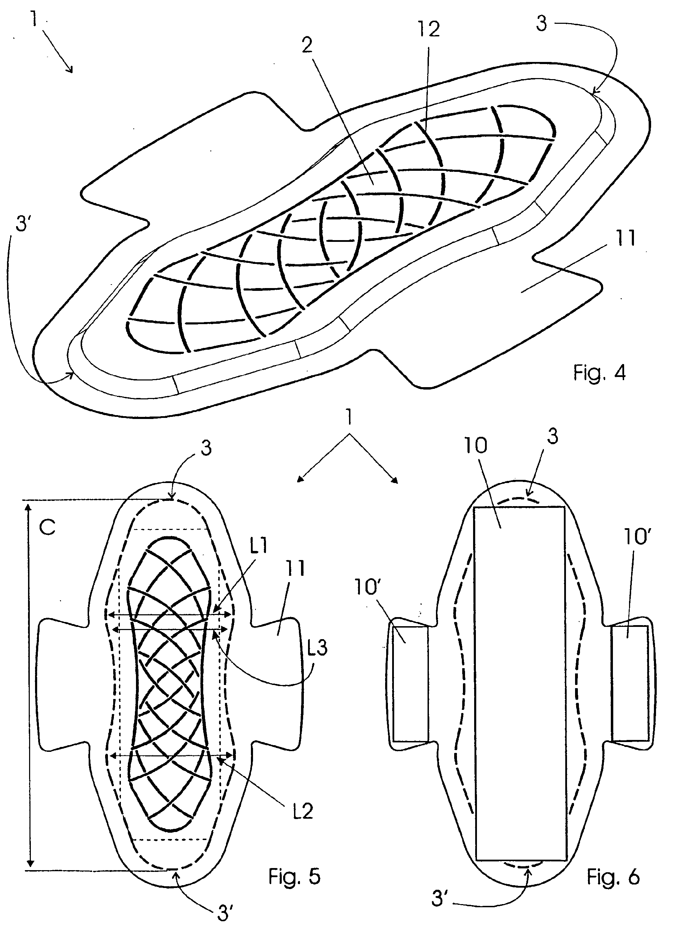

In an embodiment, the sanitary napkin has a plurality of formations of different grooves, which are spaced from one another and intercept each other. An example of such an embodiment is shown in Figures 4 and 5. With reference to these figures, the absorbent portion 2 is provided with a plurality of arcuate grooves 12, which extend in a generally oblique way with respect to a longitudinal central line and extend form one side of the surface of the sanitary napkin, through the absorption center of the sanitary napkin, to the other side. This pattern not only stabilizes the sanitary napkin, but also conducts the liquid, with efficiency, simultaneously along the length of and across the breadth of the sanitary napkin. The groove may be formed in the liquid-permeable layer and / or in the transfer layer 6. In an advantageous way, the absorbent portion 2 may optionally comprise thermoplastic fibers. The employ of thermoplastic fibers help in forming a stable and permanent groove when the thermoplastic fibers are subjected to heat application. When heat is applied, the thermoplastic fibers tend to fuse with each other to form a more rigid structure, so that the original shape of the grooves is maintained during use and with the passage of time. Conveniently, the application of heat may be incorporated into the relief process.

It is possible to use the absorbent portion 2 of the present invention in other absorbent products such as diapers for newly-born badies or for geriatric use, or other similar absorbent products.

Once a preferred embodiment has been described, it should be understood that the scope of the present invention embraces other possible variations, being only limited by the contents of the accompanying claims, which include the possible equivalents.

## Claims

1. A sanitary napkin adapted to be used in the crotch of an undergarment of a wearer for absorption of bodily exudate, comprising an absorbent portion (2) provided with a first transverse end (3) and a second opposite transverse end (3'), which define a length (C) between them, a first longitudinal side edge and a second opposite longitudinal side edge, which define a breadth between them, the breadth varying along the length of the absorbent portion (2), the napkin being **characterized in that**:
(i) a first breadth (LI) is located between 1/5 and less than ½ of the length of the absorbent portion (2), as measured from the first transverse end, the first breadth (L1) being larger than any breadth of the absorbent portion (2) situated between the first transverse end (3) and the first breadth (L1);
(ii) a second breadth (L2) located substantially at 1/5 to less than ½ of the length of the absorbent portion (2), measured from the second transverse end (3'), the second breadth (L2) being smaller than or equal to the first breadth (L1);
(iii) a central region intermediate between the first breadth and the second breadth, the central region having a maximum breadth (L3) that is smaller than or equal to the first breadth and
**characterized in that** the absorbent portion (2) has a plurality of elongate grooves (12), each groove (12) being spaced from an adjacent groove and extending
obliquely with respect to a longitudinal axis from one side of the sanitary napkin (1) to the opposite side of the sanitary napkin (1).

2. A sanitary napkin according to claim 1, **characterized in that** the first breadth (L1) is located at 1/3 of the length of the absorbent portion (2), measured from the first transverse end (3).

3. A sanitary napkin according to claim 1, **characterized in that** the central region has a maximum breadth that is smaller than the first breadth (L1), and the second breadth (L2) is smaller than the first breadth (L1).

4. A sanitary napkin according to claim 1, **characterized in that** the breadth of the absorbent portion (2) increases linearly between the first transverse end (3) and the first breadth (L1).

5. A sanitary napkin according to claim 1, **characterized in that** the first breadth (L1) and the second breadth (L2) are substantially equal, and the longitudinal side edges of the absorbent portion (2) are substantially curved in the central region, and the absorbent portion (2) is substantially hourglass-shaped.

6. A sanitary napkin according to claim 1, **characterized in that** the central region has a maximum breadth that is smaller than the first breadth (L1), the second breadth (L2) is smaller than the first breadth (L1), and the first longitudinal side edge and the second opposite longitudinal side edge converge to each other, as they approach the second transverse end (3').

7. A sanitary napkin according to claim 1, **characterized in that** the second breadth (L2) is larger than any breadth of the absorbent portion (2) located between the second transverse end (3') and the second breadth (L2), and the second breadth (L2) is substantially equal to the first breadth (L1).

## Patentansprüche

1. Hygienebinde, die dazu ausgelegt ist, in dem Zwickel eines Unterwäschestücks eines Trägers zur Absorption von Körperexsudat verwendet zu werden, mit einem absorbierenden Teil (2), das mit einem ersten quer verlaufenden Ende (3) und einem zweiten gegenüber liegenden quer verlaufenden Ende (3'), die zwischen sich eine Länge (C) definieren, einer ersten in Längsrichtung verlaufenden Seitenkante und einer zweiten gegenüber liegenden in Längsrichtung verlaufenden Seitenkante, die eine Breite zwischen sich definieren, wobei sich die Breite entlang der Länge des absorbierenden Teiles (2) ändert, versehen ist, wobei die Binde **dadurch gekennzeichnet ist, dass**:
(i) eine erste Breite (L1) sich zwischen 1/5 und weniger als ½ der Länge des absorbierenden Teils (2) befindet, von dem ersten quer verlaufenden Ende her gemessen, wobei die erste Breite (L1) größer ist als irgendeine Breite des absorbierenden Teiles (2), die zwischen dem ersten quer verlaufenden Ende (3) und der ersten Breite (L1) liegt;
(ii) eine zweite Breite (L2) sich im Wesentlichen bei 1/5 bis weniger als ½ der Länge des absorbierenden Teils (2) befindet, von dem zweiten quer verlaufenden Ende (3') her gemessen, wobei die zweite Breite (L2) kleiner als oder gleich der ersten Breite (L1) ist;
(iik) ein mittlerer Bereich zwischen der ersten Breite und der zweiten Breite angeordnet ist, wobei der mittlere Bereich eine maximale Breite (L3) hat, die kleiner oder gleich der ersten Breite ist, und
**dadurch gekennzeichnet, dass** der absorbierende Teil (2) eine Vielzahl länglicher Rinnen (12) hat, wobei jede Rinne (12) von einer benachbarten Rinne beabstandet ist und sich quer verlaufend mit Bezug auf seine Längsachse von einer Seite der Hygienebinde (1) zu der gegenüber liegenden Seite der Hygienebinde (1) erstreckt.

2. Hygienebinde nach Anspruch 1, **dadurch gekennzeichnet, dass** sich die erste Breite (L1) bei 1/3 der Länge des absorbierenden Teils (2) befindet, von dem ersten quer verlaufenden Ende (3) her gemessen.

3. Hygienebinde nach Anspruch 1, **dadurch gekennzeichnet, dass** der mittlere Bereich eine maximale Breite hat, die kleiner ist als die erste Breite (L1), und die zweite Breite (L2) kleiner als die erste Breite (L1) ist.

4. Hygienebinde nach Anspruch 1, **dadurch gekennzeichnet, dass** die Breite des absorbierenden Teiles (2) linear zwischen dem ersten quer verlaufenden Ende (3) und der ersten Breite (L1) anwächst.

5. Hygienebinde nach Anspruch 1, **dadurch gekennzeichnet, dass** die erste Breite (L1) und die zweite Breite (L2) im Wesentlichen gleich sind und die längs verlaufenden Seitenkanten des absorbierenden Teiles (2) in dem mittleren Bereich im Wesentlichen gekrümmt sind und der absorbierende Teil (2) im Wesentlichen die Form einer Sanduhr hat.

6. Hygienebinde nach Anspruch 1, **dadurch gekennzeichnet, dass** der mittlere Bereich eine maximale Breite hat, die kleiner ist als die erste Breite (L1), dass die zweite Breite (L2) kleiner ist als die erste Breite (L1) und dass die erste längs verlaufende Seitenkante und die zweite gegenüber liegende längs verlaufende Seitenkante aufeinander zu laufen, wenn sie sich an das zweite quer verlaufende Ende (3') annähern.

7. Hygienebinde nach Anspruch 1, **dadurch gekennzeichnet, dass** die zweite Breite (L2) größer ist als irgendeine Breite des absorbierenden Teiles (2), die sich zwischen dem zweiten quer verlaufenden Ende (3') und der zweiten Breite (L2) befindet, und die zweite Breite (L2) im Wesentlichen gleich der ersten Breite (L1) ist.

## Revendications

1. Serviette hygiénique adaptée pour être utilisée au niveau de l'entre-jambes d'un sous-vêtement d'une utilisatrice pour l'absorption de l'exsudat corporel, comprenant une partie absorbante (2) prévue avec une première extrémité transversale (3) et une seconde extrémité transversale opposée (3'), qui définissent une longueur (C) entre elles, un premier bord latéral longitudinal et un second bord latéral longitudinal opposé, qui définissent une largeur entre eux, la largeur variant le long de la longueur de la partie absorbante (2), la serviette étant **caractérisée en ce que** :
(i) une première largeur (L1) est située entre 1/5 et moins de ½ de la longueur de la partie absorbante (2), telle que mesurée à partir de la première extrémité transversale, la première largeur (L1) étant plus grande que n'importe quelle largeur de la partie absorbante (2) située entre la première extrémité transversale (3) et la première largeur (L1) ;
(ii) une seconde largeur (L2) située sensiblement à 1/5 jusqu'à moins de ½ de la longueur de la partie absorbante (2), mesurée à partir de la seconde extrémité transversale (3'), la seconde largeur (L2) étant plus petite ou égale à la première largeur (L1) ;
(iii) une région centrale intermédiaire entre la première largeur et la seconde largeur, la région centrale ayant une largeur maximum (L3) qui est plus petite ou égale à la première largeur, et
**caractérisée en ce que** la partie absorbante (2) a une pluralité de rainures allongées (12), chaque rainure (12) étant espacée d'une rainure adjacente et s'étendant de manière oblique par rapport à un axe longitudinal d'un côté de la serviette hygiénique (1) jusqu'au côté opposé de la serviette hygiénique (1).

2. Serviette hygiénique selon la revendication 1, **caractérisée en ce que** la première largeur (L1) est située à 1/3 de la longueur de la partie absorbante (2), mesurée à partir de la première extrémité transversale (3).

3. Serviette hygiénique selon la revendication 1, **caractérisée en ce que** la région centrale a une largeur maximum qui est plus petite que la première largeur (L1), et la seconde largeur (L2) est plus petite que la première largeur (L1).

4. Serviette hygiénique selon la revendication 1, **caractérisée en ce que** la largeur de la partie absorbante (2) augmente de manière linéaire entre la première extrémité transversale (3) et la première largeur (L1).

5. Serviette hygiénique selon la revendication 1, **caractérisée en ce que** la première largeur (L1) et la seconde largeur (L2) sont sensiblement identiques et les bords latéraux longitudinaux de la partie absorbante (2) sont sensiblement incurvés dans la région centrale, et la partie absorbante (2) a sensiblement la forme d'un sablier.

6. Serviette hygiénique selon la revendication 1, **caractérisée en ce que** la région centrale a une largeur maximum qui est plus petite que la première largeur (L1), la seconde largeur (L2) est plus petite que la première largeur (L1), et le premier bord latéral longitudinal et le second bord latéral longitudinal opposé convergent l'un vers l'autre, lorsqu'ils se rapprochent de la seconde extrémité transversale (3').

7. Serviette hygiénique selon la revendication 1, **caractérisée en ce que** la seconde largeur (L2) est plus large que n'importe quelle largeur de la partie absorbante (2) située entre la seconde extrémité transversale (3') et la seconde largeur (L2) et la seconde largeur (L2) est sensiblement identique à la première largeur (L1).
